# FASCICULE DE BREVET EUROPEEN

(11) **EP 4 225 410 B1**
(45) Date de publication et mention de la délivrance du brevet: **02.10.2024**
(21) Numéro de dépôt: 21799311.2
(22) Date de dépôt: 04.10.2021
(51) Int. Cl.: B65D 83/38, G01F 11/36, G01F 15/00, A61M 15/00, B65D 83/54

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 06.10.2020 FR 2010192
(43) Date de publication de la demande: 16.08.2023
(73) Titulaire: APTAR France SAS, 27110 Le Neubourg (FR)
(72) Inventeur: ADAM, Fabien, 27930 AVIRON (FR); BAILLIE, Mickaël, 27400 ACQUIGNY (FR)
(74) Mandataire: CAPRI
(86) Numéro de dépôt international: PCT/FR2021/051707
(87) Numéro de publication internationale: WO 2022/074316

(56) Documents cités:
- US-A1- 2005 081 846
- US-B1- 10 589 052
- US-B2- 10 556 072

## Description

La présente invention concerne un dispositif de distribution de produit fluide, et plus particulièrement un dispositif d'inhalation orale du type aérosol.

Les dispositifs d'inhalation orale sont bien connus dans l'état de la technique. L'avantage principal de ce type de dispositif est que la distribution du produit est réalisée avec un gaz propulseur pour garantir une bonne distribution du produit dans les voies respiratoires. En général, ces dispositifs comportent une valve doseuse adaptée à distribuer la même dose de produit fluide à chaque actionnement. De plus, chaque dose est distribuée de la même manière, sans possibilité de moduler les paramètres de distribution.

Les documents US10589052B1, US2019167939A1 et US10556072B2 décrivent l'utilisation d'une microvalve commandée électroniquement pour améliorer et garantir la précision de dosage d'une valve doseuse à chaque actionnement. Le document US2005081846 décrit également un dispositif de l'état de la technique.

La présente invention a pour but de fournir un dispositif de distribution de produit fluide qui ne reproduit pas les inconvénients susmentionnés.

La présente invention a aussi pour but de fournir un dispositif de distribution de produit fluide qui améliore la fiabilité de fonctionnement, en garantissant un actionnement efficace à chaque inhalation.

La présente invention a aussi pour but de fournir un dispositif de distribution de produit fluide qui permet de modifier les caractéristiques et/ou paramètres de dosage et/ou distribution à chaque actionnement.

La présente invention a également pour but de fournir un dispositif de distribution de produit fluide qui soit simple et peu coûteux à fabriquer et à assembler.

La présente a donc pour objet un dispositif de distribution de produit fluide comportant un corps pourvu d'un embout buccal, un réservoir de produit contenant du produit fluide et un gaz propulseur étant monté axialement coulissant dans ledit corps, une valve comportant une soupape étant assemblée sur ledit réservoir pour distribuer ledit produit fluide, ladite soupape étant déplaçable entre une position de repos et une position d'actionnement, la sortie de ladite soupape étant reliée audit embout buccal par un canal, ledit dispositif comportant en outre une unité électronique, dans lequel:
- ladite valve est une valve continue, dans laquelle, en position d'actionnement de la soupape, le réservoir est relié en permanence à la sortie de ladite soupape,
- ledit corps comporte un couvercle déplaçable entre une position ouverte et une position fermée, dans laquelle il sollicite ladite soupape dans sa position d'actionnement,
- ledit dispositif comporte un système de dosage et de distribution commandé par ladite unité électronique, ledit système de dosage et de distribution comportant un organe obturateur déplaçable entre une position de fermeture, dans laquelle il ferme ledit canal, et une position d'ouverture, dans laquelle il ouvre ledit canal, ledit organe obturateur étant au repos en position de fermeture et étant, lors de l'actionnement du dispositif, déplacé dans sa position d'ouverture pendant un temps prédéterminable, pour réaliser la distribution d'une dose de produit fluide.

Avantageusement, ledit couvercle est monté pivotant sur ledit corps autour d'un axe de pivotement entre ses positions ouverte et fermée, une projection axiale étant prévue dans le fond dudit couvercle, ladite projection axiale coopérant en position fermée dudit couvercle avec un fond dudit réservoir pour déplacer axialement ladite soupape en position d'actionnement.

Avantageusement, des moyens de fixation amovibles sont prévus pour fixer ledit couvercle sur ledit corps et le maintenir en position fermée.

Avantageusement, ladite unité électronique stocke des caractéristiques et/ou paramètres de dosage et/ou de distribution sur la base desquels est commandé ledit système de dosage et de distribution.

Avantageusement, lesdits caractéristiques et/ou paramètres de dosage et/ou de distribution sont modifiables.

Avantageusement, lesdits caractéristiques et/ou paramètres de dosage et/ou de distribution comprennent:
- le volume de la dose distribuée,
- la durée de la distribution,
- le débit de fluide lors de la distribution.

Avantageusement, ledit organe obturateur est commandé par un moteur ou une électrovanne commandé(e) par ladite unité électronique.

Avantageusement, ledit organe obturateur est un piston.

Avantageusement, ladite unité électronique comporte des moyens d'identification de réservoir, de sorte que lors de la mise en place d'un réservoir dans ledit corps, ladite unité électronique identifie ledit réservoir et son contenu, et adapte les caractéristiques et paramètres de dosage en fonction du type de réservoir.

Avantageusement, ladite unité électronique comporte un écran.

Avantageusement, ladite unité électronique comporte un accéléromètre pour vérifier que l'utilisateur a bien secoué le dispositif avant utilisation.

Avantageusement, ladite unité électronique comporte un capteur de position pour vérifier la position du dispositif au moment de l'utilisation.

Avantageusement, ladite unité électronique comporte un compteur de dose pour indiquer à l'utilisateur le nombre de doses distribuées ou restant à distribuer dans le réservoir.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique de côté d'un dispositif de distribution de produit fluide selon un mode de réalisation avantageux, avant assemblage du réservoir dans le corps,
la figure 2 est une vue schématique en section transversale du dispositif de la figure 1, après assemblage du réservoir dans le corps et avant fermeture du capot,
la figure 3 est une vue schématique similaire à celle de la figure 2, en cours de fermeture du capot,
la figure 4 est une vue schématique similaire à celle de la figure 3, après fermeture du capot,
la figure 5 est une autre vue schématique en section transversale du dispositif de la figure 1, après assemblage du réservoir dans le corps et avant fermeture du capot,
la figure 6 est une vue similaire à celle de la figure 5, après fermeture du capot et avant actionnement du système de distribution, et
la figure 7 est une vue similaire à celle de la figure 6, en cours d'actionnement du système de distribution.

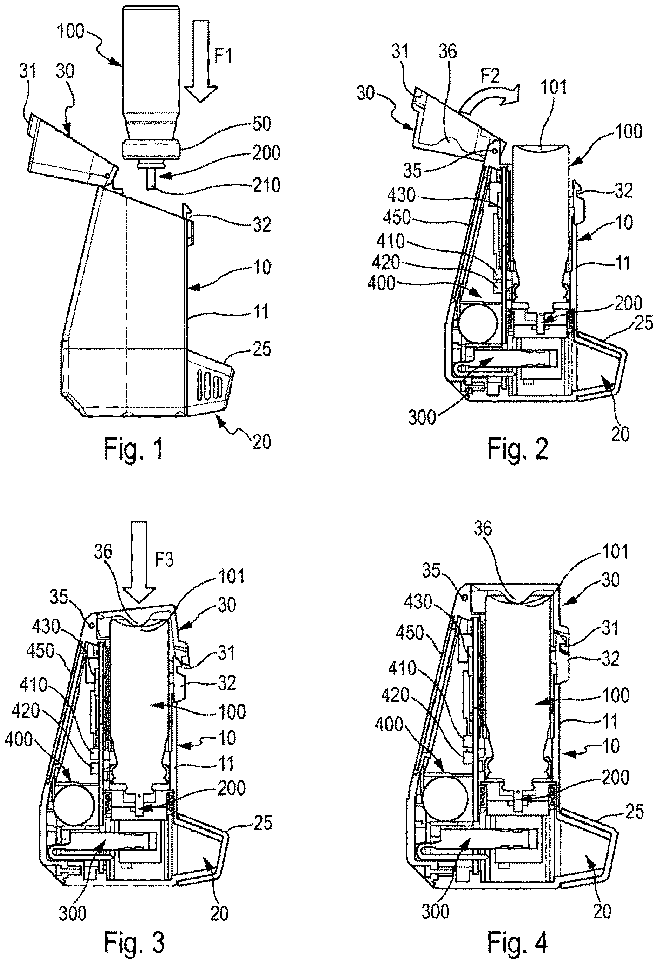

Dans la description, le terme "axial" se réfère à l'axe central vertical A de la valve représenté sur la figure 1. Les termes "proximal" et "distal" se réfèrent à l'embout buccal.

Les figures représentent un mode de réalisation avantageux de la technologie mais il est entendu que l'une ou plusieurs des pièces constitutives décrites ci-après pourrai(en)t être réalisée(s) différemment tout en procurant des fonctions similaires voire identiques.

De manière connue, un dispositif de distribution de produit fluide comporte un corps 10 pourvu d'un embout buccal 20, un réservoir de produit 100 contenant du produit fluide et une valve 200. La présente invention prévoit en outre un système de dosage et de distribution 300 d'une dose de produit fluide à chaque actionnement commandé par une unité électronique 400.

Le corps 10 peut être réalisé en une seule pièce ou en plusieurs pièces assemblées les unes aux autres. Il comporte une partie de corps cylindrique 11 recevant le réservoir 100.

Un couvercle 30 est prévu pour coopérer en position fermée avec le fond 101 du réservoir 100. Avantageusement, le couvercle 30 est monté pivotant sur le corps 10 autour d'un axe de pivotement 35 entre une position ouverte et une position fermée. Dans la position ouverte, visible sur les figures 1, 2 et 5, le réservoir 100 peut être inséré ou retiré du corps 10. Dans la position fermée, visible sur les figures 4, 6 et 7, une projection axiale 36 prévue dans le fond du couvercle 30 coopère avec le fond 101 du réservoir 100 pour déplacer axialement ledit réservoir 100 dans le corps 10.

Des moyens de fixation amovibles sont de préférence prévus pour fixer le couvercle 30 sur le corps 10 et le maintenir en position fermée. Ces moyens de fixation peuvent comprendre un crochet 32 solidaire du corps 10 coopérant avec un embout d'encliquetage 31 du couvercle qui est complémentaire du crochet 32, pour réaliser une fixation amovible, par exemple par encliquetage. D'autres moyens de fixation sont envisageables.

L'embout buccal 20 définit un orifice de distribution à travers lequel l'utilisateur va inhaler lors de l'utilisation du dispositif. L'embout buccal 20 peut être réalisé d'une pièce avec le corps 10. Un capot de protection amovible 25 peut être prévu sur ledit embout buccal 20, notamment lors des périodes de stockage, que l'utilisateur va retirer avant utilisation.

Le corps 10 contient un réservoir 100, contenant le produit à distribuer et un gaz propulseur, tel qu'un gaz du type HFA, une valve 200 étant montée sur ledit réservoir 100 pour distribuer le produit. La valve 200 comporte un corps de valve (non représenté) et une soupape 210 axialement déplaçable lors de l'actionnement par rapport audit corps de valve, et donc par rapport audit réservoir 100, entre une position de repos et une positon d'actionnement.

Selon l'invention, cette valve 200 est une valve continue, c'est-à-dire une valve dans laquelle le contenu du réservoir 100 est relié en permanence avec la sortie de la soupape 210 lorsque celle-ci est en position d'actionnement, en étant enfoncée axialement dans le corps de valve.

Cette valve 200 peut être d'un type quelconque approprié. Elle est fixée au réservoir 100 par un élément de fixation 50, de préférence une capsule sertie, de préférence avec interposition d'un joint de col (non représenté).

La présente invention ne nécessite donc pas de valve doseuse, beaucoup plus complexe et donc coûteuse à fabriquer et à assembler qu'une valve continue.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale, qui est la position d'actionnement.

Lorsque le réservoir 100 est inséré dans le corps 10 et que le couvercle 30 est en position fermée, la soupape 210 est déplacée dans sa position d'actionnement, de sorte que la valve 200 est ouverte en permanence.

L'orifice de sortie de la soupape 210 de ladite valve 200 est relié via un canal 15 audit embout buccal 20, à travers lequel l'utilisateur inhale le produit distribué.

Le système de dosage et de distribution 300 est adapté à sélectivement fermer ou ouvrir ledit canal 15. Au repos, le canal 15 est fermé, et lors de l'actionnement par l'utilisateur, le canal 15 est ouvert pendant une durée prédéterminée, générant la distribution d'une dose de produit fluide.

Ce système de dosage et de distribution 300 est commandé par une unité électronique 400 qui contient les caractéristiques et paramètres de dosage et/ou de distribution prédéterminables. Ces caractéristiques et paramètres peuvent être prédéterminés et/ou modifiés, soit par l'utilisateur, soit uniquement par du personnel autorisé. Ces modifications et réglages peuvent se faire soit directement sur le dispositif, par exemple via un écran 450 de l'unité électronique 400, soit à distance via une liaison sans fil.

Le système de dosage et de distribution 300 comporte un organe obturateur 310 déplaçable entre une position d'ouverture et une position de fermeture du canal 15. Le déplacement de l'organe obturateur 310 est commandé par un moteur ou une électrovanne commandé(e) par l'unité électronique 400.

Dans l'exemple représenté, l'organe obturateur 310 est un piston, mais il pourrait être réalisé différemment. La suite de la description sera faite en référence à un piston.

Un bouton d'actionnement peut être prévu pour permettre à l'utilisateur de déclencher la distribution d'une dose. Ce bouton peut être d'une forme quelconque, par exemple formé sur le corps 10, ou en variante être intégré à un écran 450 de l'unité électronique 400. Son actionnement par l'utilisateur déclenche l'unité électronique 400 qui va alors piloter le système de dosage et de distribution 300 conformément aux caractéristiques et paramètres de dosage et/ou de distribution stockées dans l'unité électronique 400. Avantageusement, l'unité électronique 400 peut comporter des moyens d'identification de l'utilisateur, par exemple par empreinte digitale ou reconnaissance faciale, notamment pour éviter un actionnement non autorisé du dispositif.

Ainsi, le dosage n'est plus défini par la valve 200, mais uniquement par le système de dosage et de distribution 300. Ceci permet de programmer ou prévoir des variations de dosages selon les besoins ou les utilisateurs, et/ou de modifier le dosage, si nécessaire.

Avantageusement, l'unité électronique 400 comporte des moyens d'identification du réservoir 100, de sorte que lors de la mise en place du réservoir 100 dans le corps 10, l'unité électronique 400 identifie le réservoir et son contenu, et adapte les caractéristiques et paramètres de dosage en fonction du type de réservoir. Ainsi, pour un réservoir de type A contenant un fluide A, le temps d'ouverture du piston 310 peut être de 3 secondes, alors que pour un réservoir de type B contenant un fluide B, ce temps d'ouverture peut être de 0,5 secondes.

Les caractéristiques et paramètres du dosage et/ou de la distribution peuvent notamment comporter:
- le volume de la dose distribuée,
- la durée de la distribution, typiquement entre 0,15 et 3 secondes,
- le débit de fluide lors de la distribution.

L'unité électronique 400 comporte tous les éléments électroniques nécessaires pour commander le système de dosage et de distribution 300. En particulier, elle comporte une source d'énergie, telle qu'une batterie, une pile ou un accumulateur rechargeable, un microprocesseur programmable, une mémoire. Avantageusement, un écran 450 peut être associé à l'unité électronique 400 pour afficher des informations et permettre éventuellement une interaction avec l'utilisateur. D'autres composants peuvent également être prévus, selon les besoins. Par exemple, un accéléromètre 410 est avantageusement prévu pour vérifier que l'utilisateur a bien secoué le dispositif avant utilisation. En l'absence d'une telle action de secouage, le système de dosage et de distribution 300 reste verrouillé. De plus, on prévoit avantageusement un capteur de position 420 pour vérifier la position du dispositif au moment de l'utilisation. Si le dispositif n'est pas dans une position d'utilisation correcte, le système de dosage et de distribution 300 reste verrouillé. Un compteur de dose 430 est également avantageusement prévu, pour indiquer à l'utilisateur, par exemple via l'écran 450, le nombre de doses distribuées ou restant à distribuer dans le réservoir 100. Ces différents éléments ne sont pas décrits plus amplement ici, car il s'agit de composants électroniques classiques bien connus de l'homme du métier.

L'unité électronique 400 et ses divers composants ne sont représentés que très schématiquement sur les figures, et pourraient être réalisés et disposés différemment dans le dispositif.

Un avantage de la présente invention est de pouvoir réutiliser le corps 10, le système de dosage et de distribution 300 et l'unité électronique 400. Ainsi, seuls le réservoir 100 et la valve 200 sont jetés après utilisation. Après nettoyage du canal 15 et de l'embout buccal 20, un nouveau réservoir 100 avec sa valve 200 peut être assemblé dans le corps 10.

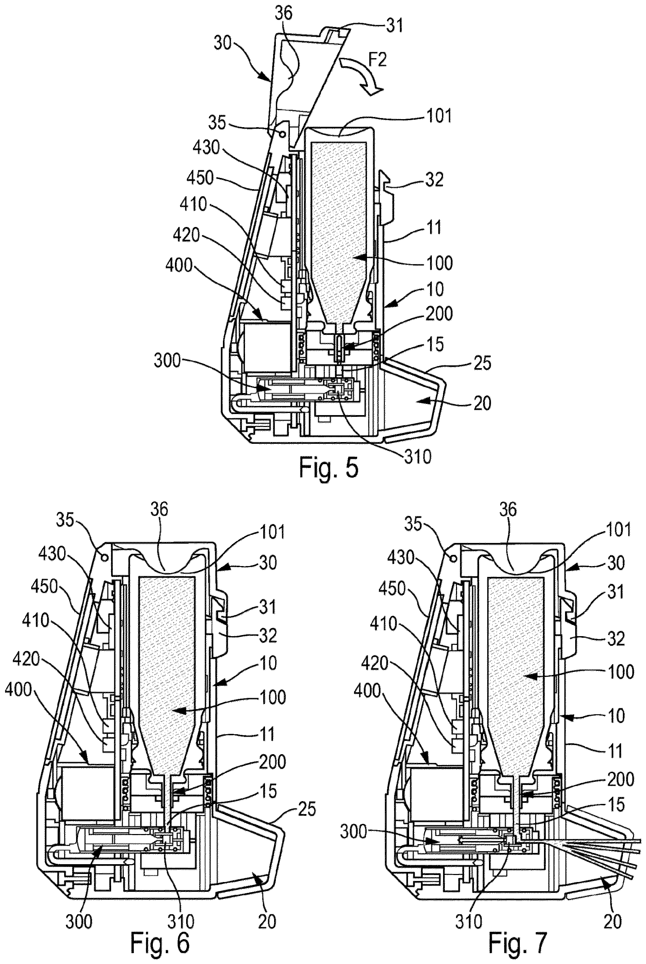

Le fonctionnement du dispositif représenté sur les figures est le suivant:
- après avoir inséré le réservoir 100 avec sa valve 200 dans le corps 10, selon la flèche F1 sur la figure 1, l'utilisateur referme le couvercle 30 selon la flèche F2 sur les figures 2 et 5;
- le verrouillage du couvercle 30 sur le corps 10 selon la flèche F3 sur la figure 3, provoque le déplacement axial du réservoir 100 par rapport à la soupape 210 de valve 200, amenant ladite valve en positon ouverte dans laquelle le réservoir est relié en permanence à la sortie de la soupape 210;
- comme le piston 310 ferme le canal 15, le produit fluide ne peut pas s'échapper hors dudit canal 15 au-delà dudit piston 310;
- lorsque l'utilisateur est prêt à utiliser le dispositif il le secoue correctement, retire le capot de protection amovible 25 de l'embout buccal 20 et place ce dernier dans sa bouche dans la bonne orientation;
- il actionne alors le dispositif, par exemple par appui sur un bouton d'actionnement, ce qui va déclencher l'unité électronique 400; celle-ci, après validation du secouage et de la bonne orientation du dispositif, va commander le système de dosage et de distribution 300 pour déplacer le piston 310 de sa position de fermeture vers sa position d'ouverture pendant un temps prédéterminé; ceci va réaliser la distribution d'une dose de produit fluide à travers l'embout buccal que l'utilisateur inhale;
- après ledit temps prédéterminé, le système de dosage et de distribution 300 va automatiquement ramener le piston 310 dans sa position de fermeture; l'utilisateur peut alors retirer le dispositif de sa bouche, et remettre le capot de protection amovible 25 sur l'embout buccal, après un éventuel nettoyage de celui-ci; le dispositif est alors prêt pour une prochaine utilisation.

La présente invention s'applique à tous types de formulations et à tous types de produits actifs contenus dans le réservoir. De même, le gaz propulseur peut être de tout type, notamment du type HFA, par exemple HFA-134a et/ou HFA-227 et/ou HFA-152a, et/ou HFO1234ze.

La présente technologie a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (10) pourvu d'un embout buccal (20), un réservoir de produit (100) contenant du produit fluide et un gaz propulseur étant monté axialement coulissant dans ledit corps (10), une valve (200) comportant une soupape (210) étant assemblée sur ledit réservoir (100) pour distribuer ledit produit fluide, ladite soupape (210) étant déplaçable entre une position de repos et une position d'actionnement, la sortie de ladite soupape (210) étant reliée audit embout buccal (20) par un canal (15), ledit dispositif comportant en outre une unité électronique (400), **caractérisé en ce que**:
- ladite valve (200) est une valve continue, dans laquelle, en position d'actionnement de la soupape (210), le réservoir (100) est relié en permanence à la sortie de ladite soupape (210),
- ledit corps (10) comporte un couvercle (30) déplaçable entre une position ouverte et une position fermée, dans laquelle il sollicite ladite soupape (210) dans sa position d'actionnement,
- ledit dispositif comporte un système de dosage et de distribution (300) commandé par ladite unité électronique (400), ledit système de dosage et de distribution (300) comportant un organe obturateur (310) déplaçable entre une position de fermeture, dans laquelle il ferme ledit canal (15), et une position d'ouverture, dans laquelle il ouvre ledit canal (15), ledit organe obturateur (310) étant au repos en position de fermeture et étant, lors de l'actionnement du dispositif, déplacé dans sa position d'ouverture pendant un temps prédéterminable, pour réaliser la distribution d'une dose de produit fluide.

2. Dispositif selon la revendication 1, dans lequel ledit couvercle (30) est monté pivotant sur ledit corps (10) autour d'un axe de pivotement (35) entre ses positions ouverte et fermée, une projection axiale (36) étant prévue dans le fond dudit couvercle (30), ladite projection axiale (36) coopérant en position fermée dudit couvercle (30) avec un fond (101) dudit réservoir (100) pour déplacer axialement ladite soupape (210) en position d'actionnement.

3. Dispositif selon la revendication 2, dans lequel des moyens de fixation amovibles (31, 32) sont prévus pour fixer ledit couvercle (30) sur ledit corps (10) et le maintenir en position fermée.

4. Dispositif selon l'une quelconque des revendications précédentes, dans lequel ladite unité électronique (400) stocke des caractéristiques et/ou paramètres de dosage et/ou de distribution sur la base desquels est commandé ledit système de dosage et de distribution (300).

5. Dispositif selon la revendication 4, dans lequel lesdits caractéristiques et/ou paramètres de dosage et/ou de distribution sont modifiables.

6. Dispositif selon l'une quelconque des revendications précédentes, dans lequel lesdits caractéristiques et/ou paramètres de dosage et/ou de distribution comprennent:
- le volume de la dose distribuée,
- la durée de la distribution,
- le débit de fluide lors de la distribution.

7. Dispositif selon l'une quelconque des revendications précédentes, dans lequel ledit organe obturateur (310) est commandé par un moteur ou une électrovanne commandé(e) par ladite unité électronique (400).

8. Dispositif selon l'une quelconque des revendications précédentes, dans lequel ledit organe obturateur (310) est un piston.

9. Dispositif selon l'une quelconque des revendications précédentes, dans lequel ladite unité électronique (400) comporte des moyens d'identification de réservoir, de sorte que lors de la mise en place d'un réservoir (100) dans ledit corps (10), ladite unité électronique (400) identifie ledit réservoir (100) et son contenu, et adapte les caractéristiques et paramètres de dosage en fonction du type de réservoir.

10. Dispositif selon l'une quelconque des revendications précédentes, dans lequel ladite unité électronique (400) comporte un écran (450).

11. Dispositif selon l'une quelconque des revendications précédentes, dans lequel ladite unité électronique (400) comporte un accéléromètre (410) pour vérifier que l'utilisateur a bien secoué le dispositif avant utilisation.

12. Dispositif selon l'une quelconque des revendications précédentes, dans lequel ladite unité électronique (400) comporte un capteur de position (420) pour vérifier la position du dispositif au moment de l'utilisation.

13. Dispositif selon l'une quelconque des revendications précédentes, dans lequel ladite unité électronique (400) comporte un compteur de dose (430) pour indiquer à l'utilisateur le nombre de doses distribuées ou restant à distribuer dans le réservoir (100).

## Patentansprüche

1. Vorrichtung zur Abgabe eines Fluidprodukts mit einem Körper (10), der mit einem Mundstück (20) versehen ist, einem Produktvorratsbehälter (100), der Fluidprodukt und ein Treibgas enthält, der axial gleitend in dem Körper (10) angebracht ist, einem Ventil (200) mit einer Ventileinrichtung (210), das an den Vorratsbehälter (100) angefügt ist, um das Fluidprodukt abzugeben, wobei die Ventileinrichtung (210) zwischen einer Ruheposition und einer Betätigungsposition bewegbar ist, wobei der Auslass der Ventileinrichtung (210) durch einen Kanal (15) mit dem Mundstück (20) verbunden ist, wobei die Vorrichtung ferner eine Elektronikeinheit (400) beinhaltet, **dadurch gekennzeichnet, dass**:
- das Ventil (200) ein Stetigventil ist, wobei in der Betätigungsposition der Ventileinrichtung (210) der Vorratsbehälter (100) permanent mit dem Auslass der Ventileinrichtung (210) verbunden ist,
- der Körper (10) einen Deckel (30) beinhaltet, der zwischen einer offenen Position und einer geschlossenen Position, in der er die Ventileinrichtung (210) in deren Betätigungsposition vorspannt, bewegbar ist,
- die Vorrichtung ein Dosier- und Abgabesystem (300) beinhaltet, das von der Elektronikeinheit (400) gesteuert wird, wobei das Dosier- und Abgabesystem (300) ein Verschlussorgan (310) beinhaltet, das zwischen einer Schließposition, in der es den Kanal (15) verschließt, und einer Öffnungsposition, in der es den Kanal (15) öffnet, bewegbar ist, wobei sich das Verschlussorgan (310) in der Schließposition in Ruhe befindet und bei der Betätigung der Vorrichtung während einer vorbestimmbaren Zeit in seine Öffnungsposition bewegt wird, um die Abgabe einer Fluidproduktdosis vorzunehmen.

2. Vorrichtung nach Anspruch 1, wobei der Deckel (30) am Körper (10) um eine Schwenkachse (35) zwischen seiner offenen und geschlossenen Position schwenkbar angebracht ist, wobei ein Axialvorsprung (36) im Boden des Deckels (30) vorgesehen ist, wobei der Axialvorsprung (36) in geschlossener Position des Deckels (30) mit einem Boden (101) des Vorratsbehälters (100) zusammenwirkt, um die Ventileinrichtung (210) in die Betätigungsposition zu bewegen.

3. Vorrichtung nach Anspruch 2, wobei lösbare Befestigungsmittel (31, 32) vorgesehen sind, um den Deckel (30) auf dem Körper (10) zu befestigen und ihn in geschlossener Position zu halten.

4. Vorrichtung nach einem der vorhergehenden Ansprüche, wobei die Elektronikeinheit (400) Dosierungs- und/oder Abgabeeigenschaften und/oder -parameter speichert, anhand derer das Dosier- und Abgabesystem (300) gesteuert wird.

5. Vorrichtung nach Anspruch 4, wobei die Dosierungs- und/oder Abgabeeigenschaften und/oder -parameter änderbar sind.

6. Vorrichtung nach einem der vorhergehenden Ansprüche, wobei die Dosierungs- und/oder Abgabeeigenschaften und/oder -parameter Folgendes umfassen:
- das Volumen der abgegebenen Dosis,
- die Dauer der Abgabe,
- der Fluiddurchsatz während der Abgabe.

7. Vorrichtung nach einem der vorhergehenden Ansprüche, wobei das Verschlussorgan (310) von einem Motor oder einem Magnetventil gesteuert wird, der bzw. das von der Elektronikeinheit (400) gesteuert wird.

8. Vorrichtung nach einem der vorhergehenden Ansprüche, wobei das Verschlussorgan (310) ein Kolben ist.

9. Vorrichtung nach einem der vorhergehenden Ansprüche, wobei die Elektronikeinheit (400) Vorratsbehälter-Identifikationsmittel beinhaltet, so dass die Elektronikeinheit (400) beim Einsetzen eines Vorratsbehälters (100) in den Körper (10) den Vorratsbehälter (100) und dessen Inhalt identifiziert und die Dosierungseigenschaften und -parameter je nach der Vorratsbehälterart anpasst.

10. Vorrichtung nach einem der vorhergehenden Ansprüche, wobei die Elektronikeinheit (400) einen Bildschirm (450) beinhaltet.

11. Vorrichtung nach einem der vorhergehenden Ansprüche, wobei die Elektronikeinheit (400) einen Beschleunigungsmesser (410) beinhaltet, um zu überprüfen, dass der Benutzer die Vorrichtung vor Gebrauch gut geschüttelt hat.

12. Vorrichtung nach einem der vorhergehenden Ansprüche, wobei die Elektronikeinheit (400) einen Positionssensor (420) beinhaltet, um die Position der Vorrichtung zum Zeitpunkt des Gebrauchs zu überprüfen.

13. Vorrichtung nach einem der vorhergehenden Ansprüche, wobei die Elektronikeinheit (400) einen Dosiszähler (430) beinhaltet, um dem Benutzer die Anzahl von abgegebenen oder im Vorratsbehälter (100) zur Abgabe verbleibenden Dosen anzuzeigen.

## Claims

1. A device for dispensing a fluid product, comprising a body (10) provided with a mouthpiece (20), a product reservoir (100) containing fluid product and a propellant gas being mounted to slide axially in said body (10), a valve (200) comprising a valve member (210) being assembled on said reservoir (100) in order to dispense said fluid product, said valve member (210) being displaceable between a rest position and an actuation position, the outlet from said valve member (210) being connected to said mouthpiece (20) by a channel (15), said device further comprising an electronic unit (400), **characterized in that**:
- said valve (200) is a continuous valve in which, in the actuation position of the valve member (210), the reservoir (100) is permanently connected to the outlet from said valve member (210),
- said body (10) comprises a cover (30) which can be displaced between an open position and a closed position in which it urges said valve member (210) into its actuation position,
- said device comprises a metering and dispensing system (300) controlled by said electronic unit (400), said metering and dispensing system (300) comprising a shutter member (310) which can be displaced between a closed position in which it closes said channel (15) and an open position in which it opens said channel (15), said shutter member (310) being at rest in the closed position and, when the device is actuated, being displaced into its open position for a predetermined time in order to dispense a dose of fluid product.

2. The device as claimed in claim 1, wherein said cover (30) is mounted to pivot on said body (10) about a pivot axis (35) between its open and closed positions, an axial projection (36) being provided in the base of said cover (30), said axial projection (36) cooperating in the closed position of said cover (30) with a base (101) of said reservoir (100) in order to axially displace said valve member (210) into the actuation position.

3. The device as claimed in claim 2, wherein releasable securing means (31, 32) are provided in order to secure said cover (30) on said body (10) and to hold it in the closed position.

4. The device as claimed in any one of the preceding claims, wherein said electronic unit (400) stores metering and/or dispensing characteristics and/or parameters on the basis of which said metering and dispensing system (300) is controlled.

5. The device as claimed in claim 4, wherein said metering and/or dispensing characteristics and/or parameters can be modified.

6. The device as claimed in any one of the preceding claims, wherein said metering and/or dispensing characteristics and/or parameters comprise:
- the volume of the dispensed dose,
- the dispensing duration,
- the flow rate of the fluid during dispensing.

7. The device as claimed in any one of the preceding claims, wherein said shutter member (310) is controlled by a motor or a solenoid valve controlled by said electronic unit (400).

8. The device as claimed in any one of the preceding claims, wherein said shutter member (310) is a piston.

9. The device as claimed in any one of the preceding claims, wherein said electronic unit (400) comprises reservoir identification means, in a manner such that when placing a reservoir (100) in said body (10), said electronic unit (400) identifies said reservoir (100) and its contents and adapts the metering characteristics and parameters as a function of the type of reservoir.

10. The device as claimed in any one of the preceding claims, wherein said electronic unit (400) comprises a screen (450).

11. The device as claimed in any one of the preceding claims, wherein said electronic unit (400) comprises an accelerometer (410) for verifying that the user has in fact shaken the device before use.

12. The device as claimed in any one of the preceding claims, wherein said electronic unit (400) comprises a position sensor (420) for verifying the position of the device at the time of use.

13. The device as claimed in any one of the preceding claims, wherein said electronic unit (400) comprises a dose counter (430) for indicating to the user the number of doses which have been dispensed or which remain to be dispensed in the reservoir (100).
